# EUROPEAN PATENT SPECIFICATION

(11) **EP 4 471 284 B1**
(45) Date of publication and mention of the grant of the patent: **25.02.2026**
(21) Application number: 23176424.2
(22) Date of filing: 31.05.2023
(51) Int. Cl.: F16C 17/02, A61L 2/00, B08B 3/00, F16C 33/10, A61L 2/18, B08B 3/02

(54) **ARM UNIT FOR A WASHING APPARATUS**
ARMEINHEIT FÜR EINE WASCHVORRICHTUNG
UNITÉ DE BRAS POUR UN APPAREIL DE LAVAGE

(43) Date of publication of application: 04.12.2024
(73) Proprietor: RESCUE Intellitech AB, 213 75 Malmö (SE)
(72) Inventor: Serruys, Robin, 233 32 Svedala (SE)
(74) Representative: Hansson Thyresson AB

(56) References cited:
- FR-A1- 2 278 985
- GB-A- 2 313 419
- US-A- 4 266 565

## Description

### TECHNICAL FIELD

The present invention relates to an arm unit for a washing apparatus, the arm unit comprising such bearing means, a washing apparatus comprising such arm unit.

### BACKGROUND

Protective clothing/equipment/gear is used in e.g., firefighting, quarrying, mining, chemical industries etc. in order to protect workers from hazardous substances. The clothing/equipment/gear is thus exposed to many substances, which may be unknown to the worker. When the work assignment is completed, the clothing/equipment/gear should be cleaned/washed/decontaminated in order not to expose the wearer/user to the substances when handling the clothing/equipment/gear.

As the clothing/equipment/gear is used in harsh environments, it may be heavily soiled and larger, coarse particles such as stone/rock material may be stuck to it. During the cleaning/washing/decontaminating process the particles are removed from the clothing/equipment/gear to be disposed of by means of the washing apparatus used for the cleaning/washing/decontaminating process.

A problem is that the material/particles, especially sharp edged particles tend to clog up or make the arms in the washing apparatus stick, and different parts of the apparatus may to get stuck or the motion of different parts become impaired. This results in an impaired washing/cleaning /decontamination of the clothing/equipment/gear, and it may also result in a disabled, broken washing apparatus.

Frequent maintenance/reparation of the washing apparatus is costly and inefficient, and having to re-wash clothing/equipment/gear which has not been properly cleaned is time consuming and inefficient.

From the above it is understood that there is room for improvements and the invention aims to solve or at least mitigate the above and other problems.

US4266565A1 discloses a dishwasher comprising a spray arm hanging from an upper rack by a self-aligning bearing structure. This document discloses an arm unit according to the preamble of claim 1.

GB2313419A discloses a bearing comprising a cylindrical shell with axial corrugations, a resilient liner, and an integral outwardly radially-directed flange.

FR2278985A1 discloses a bushing for a plain bearing comprising on one of the surfaces of its cylindrical wall at least one lubricating groove.

### SUMMARY

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of the instruments and combinations particularly pointed out in the appended claims. These and other features of the described technologies will become more fully apparent from the following description and appended claims, or may be learned by the practice of the disclosed concepts as set forth herein.

In a first aspect, an arm unit is provided, comprising a wash medium arm configured to conduct a wash medium, a hub comprising an axle, and a bearing means arranged in the wash medium arm. The bearing means is configured to be rotatably mounted to the axle of the hub. The arm unit is advantageous in that particles brought along with the wash medium and entering the bearing means of the wash medium arm are transported through the bearing means, such that the arm unit does not stick. The bearing means comprises a hub portion, and a head portion, and a bore is provided, which extends through the hub portion and the head portion. At least one first groove is provided along the bore. The groove comprises a first end portion and a second end portion. This bearing means is advantageous in that the groove provided along the bore facilitates the removal of particles having entered the bearing means. The particles may cause the bearing means to stick as they enter the bore, but with groove, the particles are led from one end of the groove towards the other without clogging the bore. The first groove facilitates transport of particles in a vertical direction of the bearing means. The bearing means is provided for a wash medium arm of a washing apparatus.

In one embodiment, the bearing means is provided with at least one second groove along a top surface of the head portion. The second groove extends in a radial direction outward from the bore and comprises a first end portion and a second end portion. The second groove facilitates the transport of particles in a horizontal direction of the bearing means. Particles having been led out of the bore by means of the first groove may get trapped between the head portion of the bearing means and an adjacent component, but the second groove facilitates such removal.

In one embodiment, the first end portion of the first groove is located at an opening of the bore in the hub portion, and the second end portion of the first groove is located at an opening of the bore in the head portion. The first end portion of the second groove is proximal to the bore of the bearing means, and the second end portion of the second groove is distal from the bore. The second end portion of the first groove is located adjacent the first end portion of the second groove, thus the first and second grooves together form a coherent groove. A coherent groove extending in both a vertical and a horizontal direction facilitates the transport of particles through and out of the bearing means, reducing the risk for the bearing means to stick.

In one embodiment, an angular displacement between the first end portion and the second end portion of the at least one first groove is one of: 0°; >0° and ≤360°; or >360°. The angular displacement between the first end portion and the second end portion of the at least one first groove facilitates the transport of particles as the bearing means is configured to rotate in an operating position.

In one embodiment, an angular displacement between the first end portion and the second end portion of the at least one second groove is one of: 0°; >0° and <360°; or >360°. The angular displacement between the first end portion and the second end portion of the at least one second groove facilitates the transport of particles as the bearing means is configured to rotate in an operating position.

In one embodiment, the bearing means is received in a through opening provided in the wash medium arm. This is a favourable way of mounting the bearing means to the washing arm.

In one embodiment, the diameter of the bore in the bearing means is dimensioned to form a spacing relative the axle. The spacing is preferably 0.2-3 mm, more preferred 0.5-4, and most preferred 1-2 mm. The spacing, or gap, contributes, together with the at least one first groove, to the transport of particles through the bearing means. The spacing thus contributes to a reliable operation of the arm unit.

In one embodiment, the first end portion of the first groove is located at an opening of the bore in the hub portion, and the second end portion of the first groove is located at an opening of the bore in the head portion. The first end portion of the second groove is proximal to the bore of the bearing means, and the second end portion of the second groove is distal from the bore. An angular displacement from the first end portion to the second end portion of at least one of the grooves of the bearing means extends in an opposite direction compared to the rotational direction of the wash medium arm. By an angular displacement extending in an opposite direction compared to the rotational direction facilitates the removal of particles from the bearing means.

In a second aspect, a washing apparatus for decontaminating personal protective equipment is provided. The washing apparatus comprises a housing enclosing a washing chamber, a pump unit configured to apply a pressure on a wash medium, and at least one arm unit arranged in the washing chamber. The washing apparatus is advantageous in that it can handle severely contaminated goods, since it is adapted to wash/decontaminate goods which brings along particles such as large/coarse/rough/sharply edged particles, but also fine particles such as dust/clay/mud into the apparatus, without affecting the operational reliability of the apparatus.

### BRIEF DESCRIPTION OF THE DRAWINGS

In order to best describe the manner in which the above-described embodiments are implemented, as well as define other advantages and features of the disclosure, a more particular description is provided below and is illustrated in the appended drawings. Understanding that these drawings depict only exemplary embodiments of the invention and are not therefore to be considered to be limiting in scope, the examples will be described and explained with additional specificity and detail through the use of the accompanying drawings in which:
- Fig. 1: is a front view of a washing apparatus,
- Fig. 2: is a section view of the washing apparatus in Fig. 1, along line B-B,
- Fig. 3: is a perspective view of the washing apparatus in Fig. 1,
- Fig. 4a: is a side view of a wash arm unit according to one embodiment,
- Fig. 4b: is a side section view of the wash arm unit in Fig. 4a along line B-B,
- Fig. 4c: is a detail of Fig. 4b according to circle F,
- Fig. 5: is a perspective view of a wash arm unit according to one embodiment,
- Fig. 6: is an exploded perspective side view of the wash arm unit in Fig 5,
- Fig. 7: is an exploded perspective side view of the wash arm unit in Fig 4,
- Fig. 8: is a perspective view of the wash arm unit in Figs 4 and 7,
- Fig. 9: is a top view of the wash arm unit in Figs 4 and 7-8
- Fig. 10a: is a perspective view of a bearing means according to one embodiment,
- Fig. 10b: is a side view of the bearing means in Fig. 10a,
- Fig. 10c: is a section view of the bearing means in Figs 10a-b along line C-C,
- Fig. 10d: is a top view of the bearing means in Fig. 10a,
- Fig. 10e: is a section view of the bearing means in Fig. 10d along line D-D,
- Fig. 11a: is a perspective view of a bearing means according to one embodiment,
- Fig. 11b: is a top view of the bearing means in Fig. 11a,
- Fig. 11c: is a side view of the bearing means in Fig. 11a, and
- Fig. 11d: is a section view of the bearing means in Figs 11c along line E-E.

Further, in the figures like reference characters designate like or corresponding parts throughout the several figures.

### DETAILED DESCRIPTION

Various embodiments of the disclosed methods and arrangements are discussed in detail below. While specific implementations are discussed, it should be understood that this is done for illustration purposes only. A person skilled in the relevant art will recognize that other components, configurations, and steps may be used without parting from the spirit and scope of the disclosure.

In the description and claims the word "comprise" and variations of the word, such as "comprising" and "comprises", does not exclude other elements or steps.

Hereinafter, certain embodiments will be described more fully with reference to the accompanying drawings. It will be apparent to those skilled in the art that various modifications and variations can be made without departing from the inventive concept. Other embodiments will be apparent to those skilled in the art from consideration of the specification and practice disclosed herein. The embodiments herein are provided by way of example so that this disclosure will be thorough and complete, and will fully convey the scope of the inventive concept, and that the claims be construed as encompassing all equivalents of the present inventive concept which are apparent to those skilled in the art to which the inventive concept pertains. If nothing else is stated, different embodiments may be combined with each other.

The present application relates to cleaning/washing/decontaminating personal protective equipment or gear and/or protective clothing. The protective clothing/equipment/gear may be adapted for firefighting, mining quarrying, chemical industries etc. The protective clothing/equipment/gear will be referred to as goods in following description, but may comprise any clothing/equipment/gear/etc. in need of cleaning. The goods may be heavily soiled and thus particles, such as stone/rock material may be brought into a cleaning/washing/decontaminating process. "Particles" refer in the following to any particles that may impair the functioning and/or operational reliability of a washing apparatus, e.g., large/coarse/rough/sharply edged particles, but also fine particles such as dust that in a certain amount form e.g., clay or mud.

Traditional washing apparatuses are designed such that particles brought into the washing apparatus by the goods, and removed from the goods during the washing process, are carried by a flow of wash medium, which circulates through the different parts of the washing apparatus. Particles entering the flow of wash medium may thus get stuck in the mechanics of the apparatus, e.g., hindering movable parts from rotating/moving as intended, or making the rotation/movement incomplete.

The inventor of the current invention has thus realized that improvement of the apparatus is advantageous in order to improve the operational reliability of the washing apparatus. In the following, an improved washing apparatus will be described with reference to the accompanied figures.

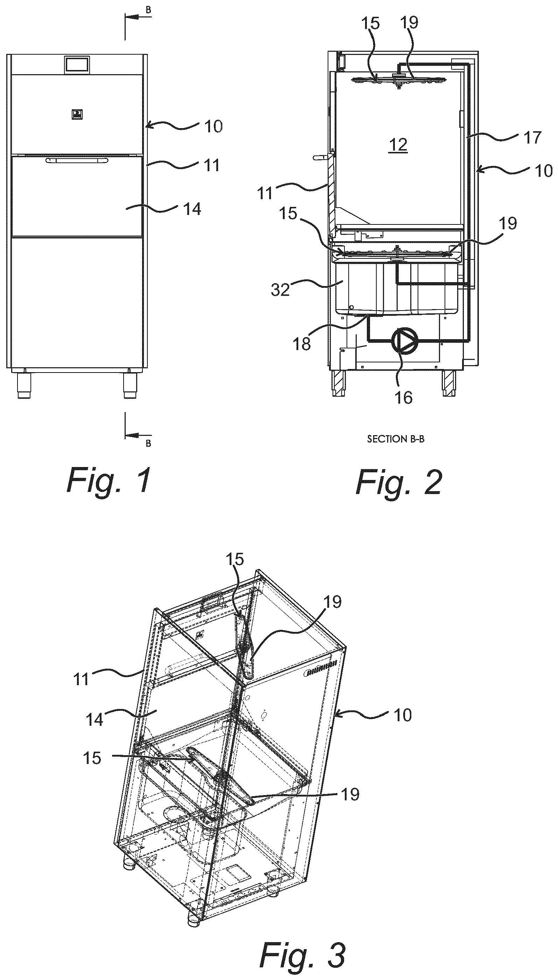

Figs 1-3 show a washing apparatus 10 comprising a housing 11 comprising a hatch or door 14, which enclose a washing chamber 12. The washing chamber 12 has at least one inlet for a wash medium, and at least one outlet 18 for the wash medium and contaminants removed from the washed goods. The washing chamber 12 is configured to receive goods to be washed.

The washing apparatus 10 comprises two arm units 15 arranged in the washing chamber 12. The arm units 15 comprise said inlets to the washing chamber 12. A first arm unit 15 is arranged in a top area of the washing chamber 12, and a second arm unit 15 is arranged in a bottom area of the washing chamber 12. Thus, when goods to be washed is arranged in the washing chamber 12, the first arm unit 15 is located above the goods, and the second arm unit 15 is located below the goods.

Each arm unit 15 comprises two rotatable arms, one wash medium arm 19 and one rinse medium arm 20. Each arm 19, 20 of each arm unit 15 is provided with openings or nozzles 21 forming the at least one inlet for a wash medium/rinse medium into the washing chamber 12. The wash medium arm 19 and the rinse medium arm 18 are hollow, in order to provide a passage for wash and rinse medium respectively to pass through them towards the nozzles 21.

The washing apparatus 10 further comprises a wash medium reservoir 32 connected to the outlet 18. A pump unit 16 is arranged in or in connection with the wash medium reservoir 32. The pump unit 16 is connected via conduits 17 to the respective first and second wash arm units 15. The pump unit 16 is configured to pump the wash medium from the wash medium reservoir 32 to the wash arm units 15, through which the wash medium is reintroduced into the washing chamber 12.

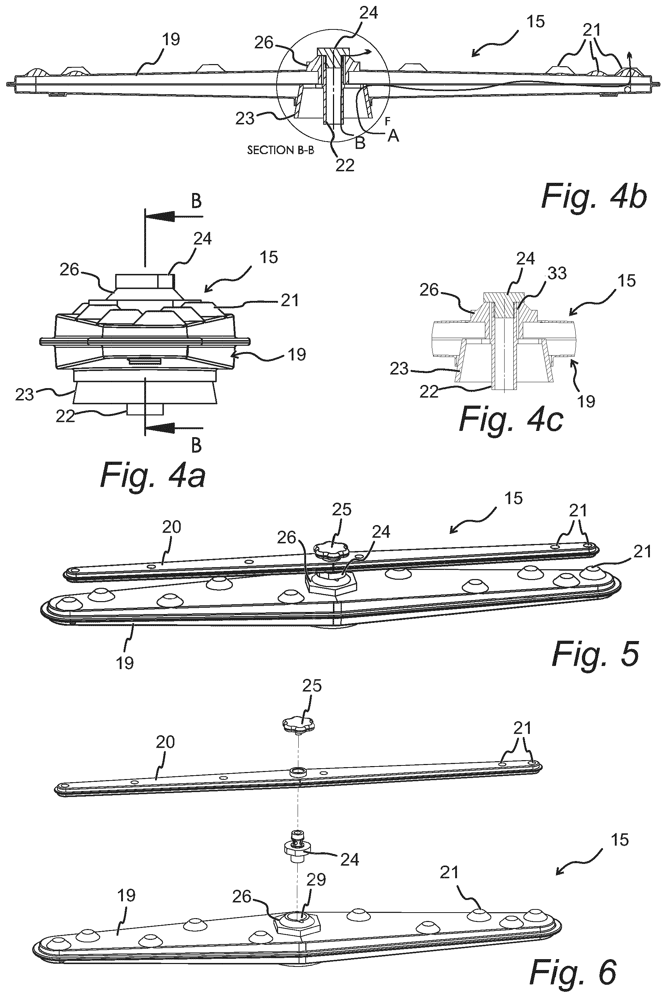

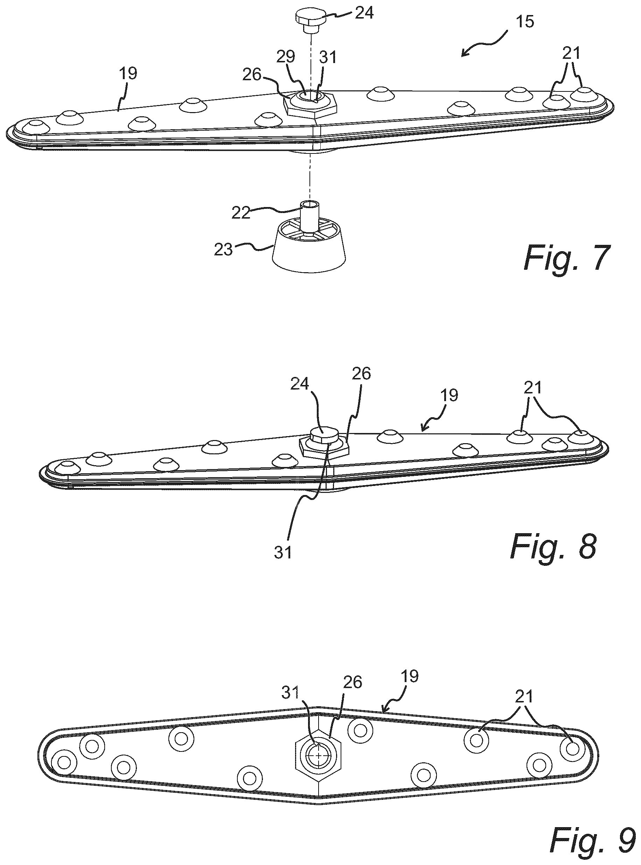

In Figs 5-6, an arm unit 15 of one embodiment is shown. The arm unit 15 comprises a wash medium arm 19, a rinse medium arm 20, a hollow axle (or spindle/shaft) 22, a hub 23, a wash medium arm bearing means 26, a rinse medium arm pivot 24 and a stop ferrule 25. In Figs 4, and 7-9, the arm unit 15 is shown without the rinse medium arm 20.

The bearing means 26 comprises a hub portion, or hub portion, 27 and a head 28. A coherent or continuous through bore 29 extends through the bearing means head 28 and the longitudinal direction of the hub portion hub portion27.

In an assembled state, as shown e.g., in Figs 5-8, the wash medium arm hub 23 is arranged on the hollow axle 22, and the wash medium arm 19 is arranged on the hub 23. The wash medium arm 19 is attached to the axle 22 by means of the bearing means 26, acting as a bearing for the rotation of the wash medium arm 19. A bearing area is thus formed between the outer surface of the axle 22 and the bore 29 of the bearing means, and between the top surface of the bearing means and the rinse medium arm pivot 24, respectively.

The hollow axle is configured to receive the rinse medium arm pivot 24, on which the rinse arm 18 is attachable. The arm unit 15 is held together by the stop ferrule 25 attached to the rinse medium arm pivot 24, e.g., by means of matching threads.

In use, rinse medium is conducted through the hollow axle 22 from a rinse medium supply (not shown) to the rinse medium arm 20, via the rinse medium arm pivot 24. The rinse medium enters the washing chamber 12 via the rinse arm nozzles 21.

Wash medium is pumped by the pump unit 16 from the wash medium reservoir 32 to the respective arm units 15 where it enters the hub 23, from which it is conducted into the inner, hollow portion of the wash medium arm 19, where it is configured to exit through the nozzles 21 and enter the washing chamber 12. See arrow A in Fig. 4b.

However, sometimes, when heavily soiled goods are washed in the washing apparatus, the goods end up not being properly washed. The impaired washing, which in the case of cleaning goods exposed to harmful and/or toxic substances, may result in harmful and/or toxic residue being left on the goods.

The inventor of the present invention realized that the impaired washing was due to the wash medium arms being hindered from rotating. The inventor further realized that this was due to a portion of the wash medium flow not entering the washing chamber 12 through the nozzles 21, but instead being pressed into the bearing area between the axle 22 and the bearing means 26. See arrow B in Fig. 4b. I.e., a portion of the wash medium passes through the bore 29 of the bearing means 26, and between an upper surface of the bearing means head 28 and a thereto abutting surface of the rinse medium arm pivot 24 in order to reach the washing chamber 12.

When material/particles are carried along with the wash medium along arrow B, into the bearing between the axle 22 and the bearing means 26, and between the upper surface of the bearing means head 28 and the abutting surface of the rinse medium arm pivot 24, there is a risk that the particles hinder the wash medium arm 19 from being able to rotate, either partly or completely, resulting in impaired washing and unreliable operation of the washing apparatus.

By realizing that this problem exists and through insightful reasoning and inventive thinking, the inventor behind this application has designed a solution where the operational reliability of the washing apparatus 10 is improved.

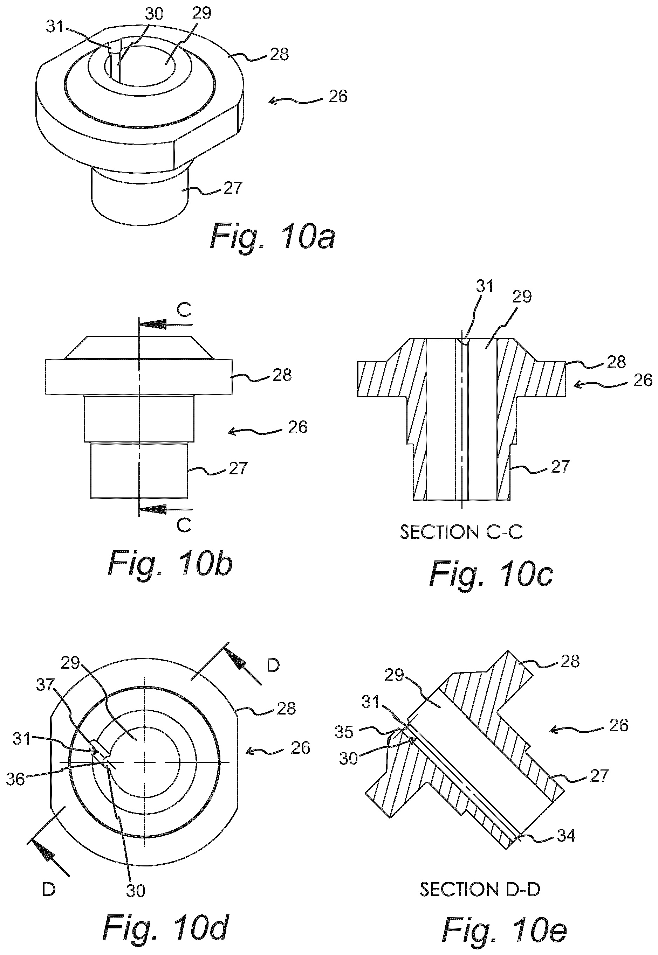

The bearing means 26, shown in detail in Figs 10-11, is provided with a first groove 30 along the continuous bore 29 extending through the hub portion 27 and head 28 of the bearing means 26.

As shown in Fig. 10e, the groove 30 has a first, lower/bottom end portion 34 located at the opening of the bore 29 in the hub portion 27 of the bearing means 26. The groove 30 has a second, top/upper portion 35 located at the opening of the bore 29 in the head 28 of the bearing means 26.

The groove 30 provides a passage between the outer surface of the axle 22 and the surface of the bore 29 of the bearing means 26 in the assembled state of the parts.

The bearing means 26 is further provided with a second groove 31 extending along the top surface of the bearing means head 28. The second groove 31 extends in a radial direction outward from the bore 29, towards a periphery of the head 28.

As shown in Fig. 10d, the groove 31 has a first, radially inner end portion 36 proximal to the bore 29 of the bearing means 26. The groove 31 has a second, radially outer end portion 37 distal from the bore 29 of the bearing means 26.

The groove 31 provides a passage between the top surface of the bearing means head 28 and the abutting surface of the rinse medium arm pivot 24 in the assembled state of the parts.

Here, "top" refers to the direction of the bearing means 26 in e.g., Figs 5-8. With this reference system, the first groove 30 may be referred to as a vertical groove, and the second groove 31 may be referred to as a horizontal groove.

The first end portion 34 of the vertical groove 30 is arranged adjacent to the first, radially inner end portion 36 of the horizontal groove 31. Thus, the two grooves 30, 31 form a coherent passage from the inner of the hub 23 to the washing chamber 12.

Additionally, in one embodiment, as shown in Fig. 4c, the diameter of the bore 29 in the bearing means 26 is dimensioned to form a spacing 33 between the surface of the bore 29 through the bearing means 26 and the outer surface of the axle 22 to form a gap between the two parts. The spacing 33 is preferably 0.2-3 mm, preferably 0.5-4, more preferably 1-2 mm.

When wash medium is pressed into the bearing area between the axle 22 and the bearing means 26, possible coarse particles conducted therewith are led through the grooves 30, 31, and thereby enters the washing chamber 12. Smaller particles may pass in the spacing 33 between the axle 22 and the bearing means 26. Thereby, the particles are transmitted into the washing chamber 12 instead of getting trapped between the axle 22 and the bearing means 26, where they may impair the rotation of the wash medium arm 19 or caused it to stop. Thus, the operational reliability of the washing apparatus is improved.

In the embodiment of the bearing means shown in Figs 10a-e, the groove 30 extends in a straight line from the bottom end portion 34 to the top end portion 35. Thus, the bottom end portion 34 of the groove 30 and the top end portion 35 are not angularly separated. In other words, the angular displacement α between the top 35 and bottom 34 ends of the groove 30 is 0°.

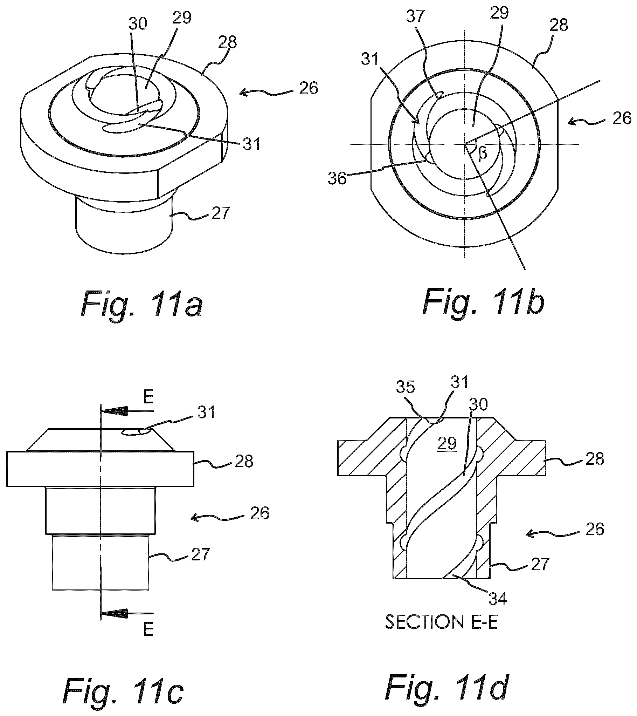

Alternatively, according to the embodiment shown in Figs 11a-d, the groove 30 extends along the bore 29 in a non-straight manner. It may e.g., extend in the manner of a helix. The bottom end portion 34 and the top end portion 35 of the groove 30 has an angular separation α of 360°. In other embodiments, the angular separation α may be more than 360°. In another embodiment, the angular separation α may be less than 360°.

The groove 31 may extend in a straight line between the first end portion 36 and the second end portion 38. Thus, the radially inner end portion 36 of the groove 31 and the radially outer end portion 37 of the groove 31 are not angularly separated. In other words, the angular displacement β between the inner 36 and outer 37 end portions of the groove 31 is separated by 0°.

Alternatively, the radially outer portion 37 of the groove 31 may have an angular displacement β in relation to the radially inner portion 36 of the groove 31, as shown in Figs 10-11. Preferably, the angular separation/displacement β between the two end portions 36, 37 of the groove 31 is 45°. Preferably, the direction of the angular displacement β of groove 31 is against, i.e., in an opposite direction compared to the direction of movement of the arm unit 15.

In one embodiment, as shown in Figs11a-d, the bearing means 26 comprises several vertical grooves 30 extending along the bore 29, and corresponding number of respectively associated horizontal grooves 31 provided in the bearing means head 28.

In one embodiment, the angular displacement α, β of the first and second grooves 30, 31, respectively, extends in a direction opposite the rotational direction of the wash medium arm 19. I.e., the helix/spiral of the helix/spiral shaped grooves 30, 31 turns in the direction opposing the rotational direction of the wash medium arm 19. This configuration is advantageous in that is facilitates transport of sand particles out of the bearing means 26.

In another embodiment, the helix/spiral direction of the helix/spiral shaped grooves 30, 31 is corresponding to the rotational direction of the wash medium arm 19.

The wash medium used to decontaminate/wash/clean the goods in the washing apparatus 10 may be one or more of water, laundry detergent, washing chemical(s), granules, dry cleaning agents, RO-water, disinfectant, soap, washing-up liquid, dish soap, dishwasher detergent, degreaser, ultrapure water.

It is beneficial to have two sets of arm units 15 in the washing apparatus 10, one providing a flow of wash medium from above, and one from below. This allows for a more thorough decontamination of the goods. As an alternative to rotatable arms, the nozzles/openings may be provided on a rotatable structure of another shape, or the structure may not be rotatable, but instead turnable back and forth. The same principles and solutions regarding the bearing area as described above are valid also for these kinds of structures.

The various embodiments described above are provided by way of illustration only and should not be construed to limit the invention. For example, the principles herein may be applied to any washing apparatus. Those skilled in the art will readily recognize various modifications and changes that may be made to the present invention without following the example embodiments and applications illustrated and described herein, and without departing from the scope of the present invention as defined in the appended claims.

For example, the washing apparatus may have only one wash arm unit.

## Claims

1. An arm unit for a washing apparatus, the arm unit comprising:
a wash medium arm (19) configured to conduct a wash medium,
a hub (23) comprising an axle (22), and
a wash medium arm bearing means (26) configured to be rotatably mounted to the axle (22) of the hub (23),
the bearing means (26) comprising a hub portion (27), and a head portion (28),
wherein a bore (29) extends through the hub portion (27) and the head portion (28),
**characterized in that**:
the bearing means (26) is arranged in the wash medium arm (19), and
at least one first groove (30) is provided along the bore (29), the groove (30) comprising a first end portion (34) and a second end portion (35).

2. The arm unit according to claim 1, wherein at least one second groove (31) is provided along a top surface of the head portion (28), extending in a radial direction outward from the bore (29), the second groove (31) comprising a first end portion (36) and a second end portion (37).

3. The arm unit according to claim 2, wherein the first end portion (34) of the first groove (30) is located at an opening of the bore (29) in the hub portion (27), and the second end portion (35) of the first groove (30) is located at an opening of the bore (29) in the head portion (28), wherein the first end portion (36) of the second groove (31) is proximal to the bore (29) of the bearing means (26), and the second end portion (37) of the second groove (31) is distal from the bore (29), and wherein the second end portion (35) of the first groove (30) is located adjacent the first end portion (36) of the second groove (31), the first (30) and second (31) grooves together forming a coherent groove.

4. The arm unit according to any one of the preceding claims, wherein an angular displacement (α) between the first end portion (34) and the second end portion (35) of the at least one first groove (30) is one of: 0°; >0° and ≤360°; or >360°.

5. The arm unit according to any one of claims 2-4, wherein an angular displacement (β) between the first end portion (36) and the second end portion (37) of the at least one second groove (31) is one of: 0°; >0° and <360°; or >360°.

6. The arm unit according to any preceding claim, wherein the bearing means (26) is received in a through opening provided in the wash medium arm (19).

7. The arm unit according to any preceding claim, wherein the diameter of the bore (29) in the bearing means (26) is dimensioned to form a spacing (33) relative the axle (22), the spacing being 0.2-3 mm, preferably 0.5-4, more preferably 1-2 mm.

8. The arm unit according to any preceding claim, wherein the first end portion (34) of the first groove (30) is located at an opening of the bore (29) in the hub portion (27), and the second end portion (35) of the first groove (30) is located at an opening of the bore (29) in the head portion (28), wherein the first end portion (36) of the second groove (31) is proximal to the bore (29) of the bearing means (26), and the second end portion (37) of the second groove (31) is distal from the bore (29), wherein an angular displacement (α, β) from the first end portion (34, 36) to the second end portion (35, 37) of at least one of the grooves (30, 31) of the bearing means (26) extends in an opposite direction as the rotational direction of the wash medium arm (19).

9. A washing apparatus (10) for decontaminating personal protective equipment, comprising:
a housing (11) enclosing a washing chamber (12),
a pump unit (16) configured to apply a pressure on a wash medium, and
at least one arm unit (15) according to any preceding claim arranged in the washing chamber (12).

## Patentansprüche

1. Armeinheit für eine Wascheinrichtung, die Armeinheit umfassend:
einen Waschmediumarm (19), der konfiguriert ist, um ein Waschmedium zu leiten,
eine Nabe (23), umfassend eine Achse (22), und
ein Waschmediumarmlagermittel (26), das konfiguriert ist, um an der Achse (22) der Nabe (23) drehbar montiert zu werden,
das Lagermittel (26) umfassend einen Nabenabschnitt (27) und einen Kopfabschnitt (28),
wobei sich eine Bohrung (29) durch den Nabenabschnitt (27) und den Kopfabschnitt (28) erstreckt,
**dadurch gekennzeichnet, dass:**
das Lagermittel (26) in dem Waschmediumarm (19) angeordnet ist und
mindestens eine erste Nut (30) entlang der Bohrung (29) bereitgestellt ist, die Nut (30) umfassend einen ersten Endabschnitt (34) und einen zweiten Endabschnitt (35).

2. Armeinheit nach Anspruch 1, wobei mindestens eine zweite Nut (31) entlang einer oberen Oberfläche des Kopfabschnitts (28) bereitgestellt ist, die sich in einer radialen Richtung nach außen von der Bohrung (29) erstreckt, die zweite Nut (31) umfassend einen ersten Endabschnitt (36) und einen zweiten Endabschnitt (37).

3. Armeinheit nach Anspruch 2, wobei der erste Endabschnitt (34) der ersten Nut (30) an einer Öffnung der Bohrung (29) in dem Nabenabschnitt (27) gelegen ist und der zweite Endabschnitt (35) der ersten Nut (30) an einer Öffnung der Bohrung (29) in dem Kopfabschnitt (28) gelegen ist, wobei sich der erste Endabschnitt (36) der zweiten Nut (31) proximal zu der Bohrung (29) des Lagermittels (26) befindet und sich der zweite Endabschnitt (37) der zweiten Nut (31) distal von der Bohrung (29) befindet, und wobei der zweite Endabschnitt (35) der ersten Nut (30) angrenzend an den ersten Endabschnitt (36) der zweiten Nut (31) gelegen ist, wobei die erste (30) und die zweite (31) Nut zusammen eine einheitliche Nut ausbilden.

4. Armeinheit nach einem der vorstehenden Ansprüche, wobei eine Winkelverschiebung (α) zwischen dem ersten Endabschnitt (34) und dem zweiten Endabschnitt (35) der mindestens einen ersten Nut (30) eines beträgt von: 0°; >0° und ≤360°; oder >360°.

5. Armeinheit nach einem der Ansprüche 2 bis 4, wobei eine Winkelverschiebung (β) zwischen dem ersten Endabschnitt (36) und dem zweiten Endabschnitt (37) der mindestens einen zweiten Nut (31) eines beträgt von: 0°; >0° und <360°; oder >360°.

6. Armeinheit nach einem der vorstehenden Ansprüche, wobei das Lagermittel (26) in einer Durchgangsöffnung, die in dem Waschmediumarm (19) bereitgestellt ist, aufgenommen wird.

7. Armeinheit nach einem der vorstehenden Ansprüche, wobei der Durchmesser der Bohrung (29) in dem Lagermittel (26) bemessen ist, um einen Abstand (33) relativ zu der Achse (22) auszubilden, wobei der Abstand 0,2-3 mm, vorzugsweise 0,5-4, mehr bevorzugt 1-2 mm, beträgt.

8. Armeinheit nach einem der vorstehenden Ansprüche, wobei der erste Endabschnitt (34) der ersten Nut (30) an einer Öffnung der Bohrung (29) in dem Nabenabschnitt (27) gelegen ist und der zweite Endabschnitt (35) der ersten Nut (30) an einer Öffnung der Bohrung (29) in dem Kopfabschnitt (28) gelegen ist, wobei sich der erste Endabschnitt (36) der zweiten Nut (31) proximal zu der Bohrung (29) des Lagermittels (26) befindet und sich der zweite Endabschnitt (37) der zweiten Nut (31) distal von der Bohrung (29) befindet, wobei sich eine Winkelverschiebung (α, β) von dem ersten Endabschnitt (34, 36) zu dem zweiten Endabschnitt (35, 37) mindestens einer der Nuten (30, 31) des Lagermittels (26) in einer entgegengesetzten Richtung zu der Drehrichtung des Waschmediumarms (19) erstreckt.

9. Wascheinrichtung (10) zum Dekontaminieren persönlicher Schutzausrüstung, umfassend:
ein Gehäuse (11), das eine Waschkammer (12) umschließt,
eine Pumpeneinheit (16), die konfiguriert ist, um einen Druck auf ein Waschmedium aufzubringen, und
mindestens eine Armeinheit (15) nach einem der vorstehenden Ansprüche, die in der Waschkammer (12) angeordnet ist.

## Revendications

1. Unité de bras pour un appareil de lavage, l'unité de bras comprenant :
un bras de milieu de lavage (19) conçu pour conduire un milieu de lavage,
un moyeu (23) comprenant un essieu (22), et
un moyen de palier de bras de milieu de lavage (26) conçu pour être monté de manière rotative sur l'axe (22) du moyeu (23),
les moyens de palier (26) comprenant une partie de moyeu (27) et une partie de tête (28),
dans lequel un alésage (29) s'étend à travers la partie de moyeu (27) et la partie de tête (28),
**caractérisée en ce que** :
les moyens de palier (26) sont agencés dans le bras de milieu de lavage (19), et
au moins une première rainure (30) est fournie le long de l'alésage (29), la rainure (30) comprenant une première partie d'extrémité (34) et une seconde partie d'extrémité (35).

2. Unité de bras selon la revendication 1, dans laquelle au moins une seconde rainure (31) est prévue le long d'une surface supérieure de la partie de tête (28), s'étendant dans une direction radiale vers l'extérieur à partir de l'alésage (29), la seconde rainure (31) comprenant une première partie d'extrémité (36) et une seconde partie d'extrémité (37).

3. Unité de bras selon la revendication 2, dans laquelle la première partie d'extrémité (34) de la première rainure (30) est située au niveau d'une ouverture de l'alésage (29) dans la partie de moyeu (27), et la seconde partie d'extrémité (35) de la première rainure (30) est située au niveau d'une ouverture de l'alésage (29) dans la partie de tête (28), dans lequel la première partie d'extrémité (36) de la seconde rainure (31) est proximale à l'alésage (29) des moyens de palier (26), et la seconde partie d'extrémité (37) de la seconde rainure (31) est distale par rapport à l'alésage (29), et dans lequel la seconde partie d'extrémité (35) de la première rainure (30) est située adjacente à la première partie d'extrémité (36) de la seconde rainure (31), la première (30) et la seconde (31) rainure formant ensemble une rainure cohérente.

4. Unité de bras selon l'une quelconque des revendications précédentes, dans laquelle un déplacement angulaire (α) entre la première partie d'extrémité (34) et la seconde partie d'extrémité (35) de l'au moins une première rainure (30) est l'un parmi : 0°; >0° et ≤360° ; ou >360°.

5. Unité de bras selon l'une quelconque des revendications 2 à 4, dans laquelle un déplacement angulaire (β) entre la première partie d'extrémité (36) et la seconde partie d'extrémité (37) de l'au moins une seconde rainure (31) est l'un parmi : 0° ; >0° et <360° ; ou >360°.

6. Unité de bras selon l'une quelconque revendication précédente, dans laquelle les moyens de palier (26) sont reçus dans une ouverture traversante prévue dans le bras de milieu de lavage (19).

7. Unité de bras selon l'une quelconque revendication précédente, dans laquelle le diamètre de l'alésage (29) dans les moyens de palier (26) sont dimensionnés pour former un espacement (33) par rapport à l'essieu (22), l'espacement étant de 0,2 à 3 mm, de préférence de 0,5 à 4, plus préférablement de 1 à 2 mm.

8. Unité de bras selon l'une quelconque revendication précédente, dans laquelle la première partie d'extrémité (34) de la première rainure (30) est située au niveau d'une ouverture de l'alésage (29) dans la partie de moyeu (27), et la seconde partie d'extrémité (35) de la première rainure (30) est située au niveau d'une ouverture de l'alésage (29) dans la partie de tête (28), dans lequel la première partie d'extrémité (36) de la seconde rainure (31) est proximale à l'alésage (29) des moyens de palier (26), et la seconde partie d'extrémité (37) de la seconde rainure (31) est distale par rapport à l'alésage (29), dans lequel un déplacement angulaire (a, P) de la première partie d'extrémité (34, 36) à la seconde partie d'extrémité (35, 37) d'au moins l'une des rainures (30, 31) des moyens de palier (26) s'étend dans une direction opposée à la direction de rotation du bras de milieu de lavage (19).

9. Appareil de lavage (10) permettant de décontaminer un équipement de protection individuelle, comprenant :
un boîtier (11) renfermant une chambre de lavage (12),
une unité de pompe (16) conçue pour appliquer une pression sur un milieu de lavage, et
au moins une unité de bras (15) selon l'une quelconque revendication précédente, agencée dans la chambre de lavage (12).
